(19) Europäisches Patentamt
European Patent Office
Office européen des brevets

(11) Publication number: **0 044 890**
**B1**

# (12) EUROPEAN PATENT SPECIFICATION

(45) Date of publication of patent specification: **28.01.87**

(51) Int. Cl.⁴: **C 07 D 213/61** // C07B39/00, B01J27/08

(21) Application number: **80302568.3**

(22) Date of filing: **28.07.80**

## (54) Process for preparing fluoropyridine compounds.

(43) Date of publication of application:
**03.02.82 Bulletin 82/05**

(45) Publication of the grant of the patent:
**28.01.87 Bulletin 87/05**

(84) Designated Contracting States:
**BE DE FR IT NL**

(56) References cited:
**US-A-3 296 269**
**US-A-3 303 197**
**US-A-4 031 100**

(73) Proprietor: **DOW CHEMICAL COMPANY LIMITED**
**Stana Place, Fairfield Ave.**
**Staines, Middlesex TW18 4SX (GB)**

(72) Inventor: **Jones, Edward Malcolm**
**8, Maple Drive South Wootton**
**Kings Lynn Norfolk (GB)**

(74) Representative: **Hardisty, David Robert et al**
**BOULT, WADE & TENNANT 27 Furnival street**
**London EC4A IPQ (GB)**

EP 0 044 890 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns an improvement in the reaction of a chlorinated pyridine compound with an alkali metal fluoride. Products of the reaction are useful as intermediates for the preparation of herbicides or pesticides.

It is known to react chloropyridines with potassium fluoride in a polar aprotic solvent in the presence of a small amount of water to effect replacement of chlorine by fluorine. Reaction of pentachloropyridine with anhydrous KF in a 1:5.9 mole ratio in sulpholane and water at 215°C has given 91% yield of 3,5-dichlorotrifluoropyridine, 6% yield of 3-chlorotetrafluoropyridine, and 3% yield of 3,4,5,6-tetrachlorofluoropyridine (GB—A—1,256,082). Reaction of pentachloropyridine with KF in a 1:11 mole ratio at 400°C for 18 hours in the absence of a solvent has given 84% yield of 3,5-dichloro-2,4,6-trifluoropyridine (J. Chem. Soc. *1964,* 3573). Reaction of pentachloropyridine with KF in a 1:9.5 mole ratio at 400°C for 5 hours in the absence of solvent has given 45% yield of 3,5-dichlorotrifluoropyridine and 19% yield of 3-chlorotetrafluoropyridine (J. Chem. Soc. *1965,* 594).

It has now been found that certain compounds are effective catalysts for the reaction of a chlorinated pyridine compound with an alkali metal fluoride and that carrying out the reaction in the presence of a small amount of one or more of these compounds permits use of less alkali metal fluoride and lower temperatures than heretofore for preparing a given desired product in good yield.

Accordingly, the desired invention provides a process for preparing a fluoropyridine compound, which process comprises reacting a chlorinated pyridine compound with a molar excess of an alkali metal fluoride in the presence of a catalytic amount of a catalyst selected from a chloride or bromide of aluminium or antimony, or a halide of the metals of the iron, nickel and copper groups, whereby at least one chloride of the chlorinated pyridine compound is replaced by fluorine. The desired product may be isolated by distallation.

The starting material may be any pyridine compound containing at least one chlorine substituent on the ring. The useful starting materials include, for example, the mono-, di-, tri-, tetra- and penta-chloropyridines, depending on the particular product or products desired. When the starting material is a polychloropyridine, the number of chlorine atoms replaced by fluorine is believed to be dependent on the temperature and time of reaction employed, the higher temperatures and longer times of reaction favouring more replacement by fluorine. The process of the invention is of special interest for the preparation of 3,5-dichloro-2,4,6-trifluoropyridine from pentachloropyridine; the 3,5-dichloro-2,6-difluoropyridine from 2,3,5,6-tetrachloropyridine and 2,6-difluoropyridine from 2,6-dichloropyridine.

The alkali metal fluoride used in the process of the present invention may be cesium, potassium or sodium fluoride. The use of potassium fluoride is preferred.

As indicated above, it is essential to use a molar excess of the alkali metal fluoride. Preferably, the amount of alkali metal fluoride employed is 10 to 100%, more preferably 10 to 50%, in excess of the theoretical amount of fluorine required to produce the desired fluoropyridine.

An essential feature of the present process is the utilization of a catalyst selected from those listed above. Preferred catalysts include $SbCl_3$, $AlCl_3$, $ZnCl_2$ and $FeCl_3$, with the latter being most preferred. In order to obtain the benefits of the present invention, it is also essential that the catalyst be employed in an amount sufficient to provide a catalytic effect. Having regard to economy and ease of carrying out the reaction, the catalyst is generally employed in an amount of from 0.5 to 10, preferably from 1 to 5, more preferably from 1 to 3, percent by weight based on the amount of the alkali metal fluoride.

For economic reasons, it is generally preferred to carry out the reaction of the process of this invention without the use of a solvent, although the reaction can be run in the presence of a solvent or reaction medium, such as sulpholane.

Although the reaction can be carried out successfully in glass equipment, glass is susceptible to attack by alkali metal fluorides, and it may be desired to employ a greater amount of the alkali metal fluoride than the preferred amounts indicated above. In view of this, it is preferred to carry out the reaction in equipment in which the parts contacted by the reactants are constructed of material that is inert to alkali metal fluorides, such as stainless steel, nickel or Hastelloy C.

The conditions under which the reaction is carried out are such that the reactants are in their liquid or gaseous states. Below 150°C there is a tendency for the reactant to be in their solid states, and accordingly it is preferred to carry out the reaction at a temperature within the range of from 150° to 400°C, more preferably at a temperature within the range of from 250° to 350°C and most preferably within the range of 250° to 300°C. The reaction is preferably carried out in sealed vessels necessitating elevated pressures which are dependent on the temperatures used and the number of moles of starting material. Typically at 300°C the pressure is in the range of from 50 to 200 psig (4.4—14.6 atm).

A particularly preferred process of the present invention comprises reacting in a sealed inert vessel pentachloropyridine with from 3.3 to 4.5 moles of potassium fluoride per mole of the pentachloropyridine in the presence of from 1 to 3 percent by weight of $FeCl_3$ based on the amount of potassium fluoride and in the absence of a solvent at a temperature within the range of from 250° to 350°C, and recovering 3,5-dichloro-2,4,6-trifluoropyridine as the principal product.

The fluoropyridine compounds prepared according to the present invention are particularly useful as intermediates for the preparation of agriculturally-useful fluoropyridine compounds. Examples of such

agriculturally-useful fluoropyridine compounds which can be prepared employing the fluoropyridine compounds prepared as taught herein include compounds as follows.

(a) Compounds of general formula:

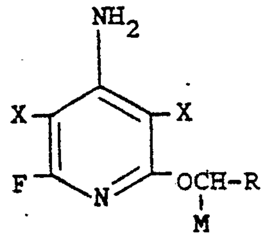

wherein each X independently represents chloro, bromo or fluoro; R represents —$CONR^3R^4$ (where $R^3$ and $R^4$ independently each represents hydrogen or alkyl containing from 1 to 8 carbon atoms), —COOH or a salt thereof, or —$COOR^5$ (where $R^5$ represents alkyl containing from 1 to 12 carbon atoms or —$(CH_2)_nOR^6$ where n represents an integer of from 2 to 4 and $R^6$ represents lower alkyl or phenyl); and M represents hydrogen or lower alkyl.

Such compounds, agriculturally-useful compositions containing them, and their uses are described and claimed in GB—A—1,418,979.

(b) A compound of formula:

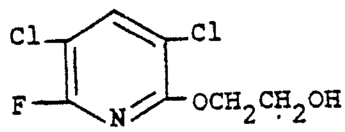

and

(c) A compound of formula:

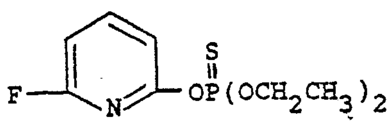

Any agriculturally-useful fluoropyridine, including those specified in (a) to (c) above, which has been prepared directly or indirectly from a fluoropyridine prepared by a process according to the present invention is within the scope of the present invention. So is an agriculturally-useful composition comprising any such agriculturally-useful fluoropyridine. Those compounds specified in (a) and (b) above are herbicidally active and the compound specified in (c) above is a nematocide.

The agriculturally-useful fluoropyridines can be prepared from the fluoropyridines prepared by the process of the present invention by any appropriate route. A typical example of one such route is as follows:

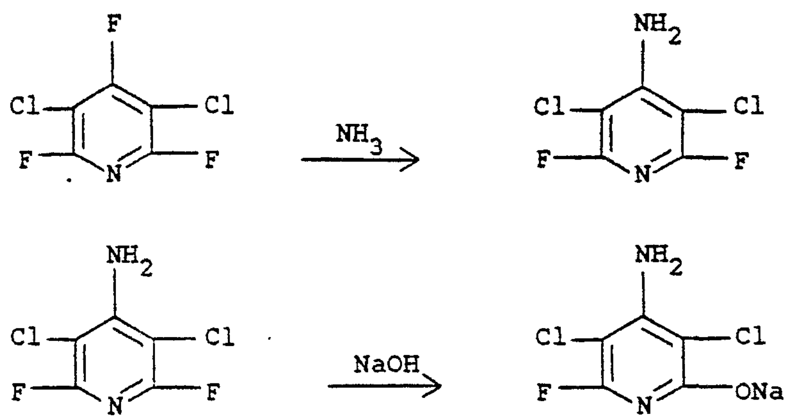

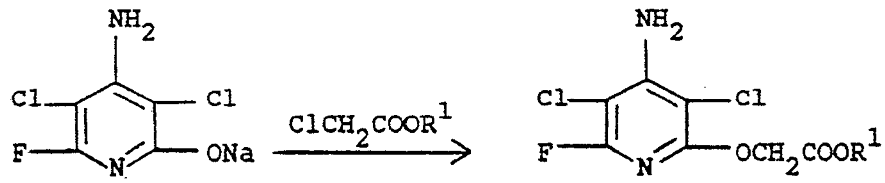

where $R^1$ is as defined above.

It will be apparent to those skilled in the art that a wide range of agriculturally-useful fluoropyridines can be prepared from a fluoropyridine which has been prepared by the process according to the present invention.

The following Examples further illustrate the present invention.

Example 1

A mixture of 100 g potassium fluoride and 1 g ferric chloride was prepared by grinding together the two components. In like manner, 2 g (0.008 mole) of pentachloropyridine were mixed with 1.87 g (0.032 mole KF) of the KF/$FeCl_3$ mixture. The mixing operations were carried out in a dry nitrogen atmosphere. The mixture of the reactants was placed in a stainless steel cylinder and the cylinder sealed. Then the cylinder and contents were heated in a furnace at 300°C for 16 hours. After cooling, the contents were extracted with chloroform and filtered. Analysis by gas chromatography indicated an 83 percent yield of 3,5-dichloro-2,4,6-trifluoropyridine and a 4 percent yield of 3-chloro-2,4,5,6-tetrafluoropyridine.

Example 2

The method of this Example was similar to that of Example 1 except as follows: The reaction mixture contained 3.74 g (0.64 mole KF) of the KF/$FeCl_3$ mixture (KF:pentachloropyridine molar ratio 8:1). The reaction vessel was glass, and the contents were heated at 340°C for 30 hours. There was obtained a 31 percent yield of 2,3,4,5-tetrachloro-6-fluoropyridine, a 57 percent yield of 2,3,5-trichloro-4,6-difluoropyridine, and an 8 percent yield of 3,5-dichloro-2,4,6-trifluoropyridine.

Example 3

The method for this Example was similar to Example 1 except as follows. The reaction mixture contained 2 g (0.008 mole) pentachloropyridine and 1.62 g (0.028 mole KF) of the KF/$FeCl_3$ mixture, and the reactants were heated at 250°C. There was obtained 2 percent yield of 2,3,4,5-tetrachloro-6-fluoropyridine, 24 percent yield of 2,3,5-trichloro-4,6-difluoropyridine and 61 percent yield of 3,5-dichloro-2,4,6-trifluoropyridine.

Example 4 (Comparative)

Example 3 was repeated except that no catalyst ($FeCl_3$) was used. There was obtained 5 percent unreacted pentachloropyridine, 38 percent yield of 2,3,4,5-tetrachloro-6-fluoropyridine, 33 percent yield of 2,3,5-trichloro-4,6-difluoropyridine and 17 percent yield of 3,5-dichloro-2,4,6-trifluoropyridine.

Example 5

The method for this Example was similar to Example 1 except as follows. The reaction mixture contained 2 g (0.0092 mole) 2,3,5,6-tetrachloropyridine and 1.62 g (0.0276 mole KF) of the KF/$FeCl_3$ mixture. There was obtained 70 percent yield of 3,5-dichloro-2,6-difluoropyridine.

Example 6 (Comparative)

Example 5 was repeated except that no catalyst ($FeCl_3$) was used. There was obtained 1 percent of unreacted 2,3,5,6-tetrachloropyridine, 22 percent yield of 2,3,5-trichloro-6-fluoropyridine and 61 percent yield of 3,5-dichloro-2,6-difluoropyridine.

**Claims**

1. Process for preparing a fluoropyridine compound which comprises reacting a chlorinated pyridine compound with a molar excess of an alkali metal fluoride characterized in that the reaction is carried out in the presence of a catalytic amount of a chloride or bromide of aluminium or antimony or a halide of the metals of the iron, nickel and copper groups, whereby at least one chlorine of the chlorinated pyridine compound is replaced by fluorine.

2. A process as claimed in Claim 1 wherein the halide is a bromide or a chloride.

3. A process as claimed in Claim 1 or Claim 2 wherein the catalyst is $SbCl_3$, $AlCl_3$, $FeCl_3$ or $ZnCl_2$.

4. A process as claimed in any one of the preceding claims wherein the amount of alkali metal fluoride used is 10 to 50% in excess of the theoretical amount of fluorine required to produce the desired fluoropyridine.

5. A process as claimed in any one of the preceding claims wherein the catalyst is employed in an amount of from 1 to 3 percent by weight based on the amount of alkali metal fluoride.

6. A process as claimed in any one of the preceding claims wherein the alkali metal fluoride employed is KF.

7. A process as claimed in any one of the preceding claims wherein reaction is carried out in the absence of a solvent.

8. A process as claimed in any one of the preceding claims wherein the reaction is carried out at a temperature within the range from 250° to 350°C.

9. A process as claimed in any one of the preceding claims wherein the chlorinated pyridine compound reactant is pentachloropyridine or 2,3,5,6-tetrachloropyridine.

**Patentansprüche**

1. Verfahren zur Herstellung einer Fluorpyridinverbindung durch Umsetzung einer chlorierten Pyridinverbindung mit einem molaren Überschuß eines Alkalimetallfluorids, dadurch gekennzeichnet, daß die Umsetzung in Gegenwart einer katalytischen Menge eines Chlorids oder Bromids von Aluminium oder Antimon oder eines Halogenids der Metalle der Eisen-, Nickel- und Kupfergruppen durchgeführt wird, wobei mindestens ein Chlor der chlorierten Pyridinverbindung durch Fluor ersetzt wird.

2. Verfahren nach Anspruch 1, dadurch gekennzeichnet, daß das Halogenid ein Bromid oder ein Chlorid ist.

3. Verfahren nach Anspruch 1 oder 2, dadurch gekennzeichnet, daß der Katalysator $SbCl_3$, $AlCl_3$, $FeCl_3$ oder $ZnCl_2$ ist.

4. Verfahren nach einem der vorhergehenden Ansprüche, dadurch gekennzeichnet, daß die Menge an verwendetem Alkalimetallfluorid der 10- bis 50 %-ige Überschuß der zur Herstellung des gewünschten Fluorpyridins erforderlichen theoretischen Fluormenge ist.

5. Verfahren nach einem der vorhergehenden Ansprüche, dadurch gekennzeichnet, daß der Katalysator in einer Menge von 1 bis 3 Gew.-%, bezogen auf die Menge Alkalimetallfluorid, verwendet wird.

6. Verfahren nach einem der vorhergehenden Ansprüche, dadurch gekennzeichnet, daß das verwendete Alkalimetallfluorid KF ist.

7. Verfahren nach einem der vorhergehenden Ansprüche, dadurch gekennzeichnet, daß die Umsetzung in Abwesenheit eines Lösungsmittels durchgeführt wird.

8. Verfahren nach einem der vorhergehenden Ansprüche, dadurch gekennzeichnet, daß die Umsetzung bei einer Temperatur im Bereich von 250°C bis 350°C durchgeführt wird.

9. Verfahren nach einem der vorhergehenden Ansprüche, dadurch gekennzeichnet, daß die als Reaktionsteilnehmer eingesetzte chlorierte Pyridinverbindung Pentachlorpyridin oder 2,3,5,6-Tetrachlorpyridin ist.

**Revendications**

1. Procédé pour la préparation d'une fluoropyridine qui consiste à faire réagir une pyridine chlorée avec un excès molaire d'un fluorure de métal alcalin, caractérisé en ce que la réaction est effectuée en présence d'une quantité catalytique d'un chlorure ou d'un bromure d'aluminium ou d'antimoine ou d'un halogénure des métaux des groupes du fer, du nickel et du cuivre, ce qui permet le remplacement d'au moins un chlore de la pyridine chlorée par du fluor.

2. Procédé selon la revendication 1, caractérisé en ce que l'halogénure est un bromure ou un chlorure.

3. Procédé selon la revendication 1 ou 2, caractérisé en ce que le catalyseur est $SbCl_3$, $AlCl_3$, $FeCl_3$ ou $ZnCl_2$.

4. Procédé selon l'une quelconque des revendications précédentes, caractérisé en ce que la quantité de fluorure de métal alcalin utilisé est de 10 à 50% en excès par rapport à la quantité théorique de fluor nécessaire pour produire la fluoropyridine déirée.

5. Procédé selon l'une quelconque des revendications précédentes, caractérisé en ce que le catalyseur est utilisé en une quantité allant de 1 à 3 pour cent en poids par rapport à la quantité de fluorure de métal alcalin.

6. Procédé selon l'une quelconque des revendications précédentes, caractérisé en ce que le fluorure de métal alcalin utilisé est KF.

7. Procédé selon l'une quelconque des revendications précédentes, caractérisé en ce que la réaction est effectuée en l'absence d'un solvant.

8. Procédé selon l'une quelconque des revendications précédentes, caractérisé en ce que la réaction est effectuée à une température comprise dans l'intervalle allant de 250° à 350°C.

9. Procédé selon l'une quelconque des revendications précédentes, caractérisé en ce que la pyridine chlorée utilisée en tant que corps réagissant est la pentachloropyridine ou la 2,3,5,6-tétrachloropyridine.